# EUROPÄISCHE PATENTSCHRIFT

(11) **EP 3 968 335 B1**
(45) Veröffentlichungstag und Bekanntmachung des Hinweises auf die Patenterteilung: **08.07.2026**
(21) Anmeldenummer: 20195297.5
(22) Anmeldetag: 09.09.2020
(51) Int. Cl.: G16H 20/13, G16H 40/63, G16H 40/67

(54) **INTERAKTIVES THERAPIEGERÄT UND VERNETZTES THERAPIESYSTEM**
INTERACTIVE THERAPY DEVICE AND NETWORKED THERAPY SYSTEM
APPAREIL THÉRAPEUTIQUE INTERACTIF ET SYSTÈME DE THÉRAPIE EN RÉSEAU

(43) Veröffentlichungstag der Anmeldung: 16.03.2022
(73) Patentinhaber: Pulmotree Medical GmbH, 80333 München (DE)
(72) Erfinder: Görstner, Pia, 80335 München (DE); Kellner, Kristian, 80804 München (DE); Krüger, Ulf, 80799 München (DE)
(74) Vertreter: Stolmár & Partner Patentanwälte PartG mbB

(56) Entgegenhaltungen:
- EP-A1- 3 375 473

## Beschreibung

Die Erfindung betrifft ein interaktives Therapiegerät sowie ein vernetztes Therapiesystem.

Als "interaktive Therapiegeräte" im Sinne dieser Erfindung werden Therapiegeräte verstanden, welche ein bestimmtes Therapieverfahren in Abhängigkeit von einer Interaktion eines Benutzers, insbesondere eines Patienten, mit dem Therapiegerät anwenden.

Ein mögliches Beispiel interaktiver Therapiegeräte sind Inhalatoren, welche zur Abgabe eines Therapeutikums in einen vom Patienten eingeatmeten Luftstroms ausgeführt sind. Bei solchen Inhalatoren ist die Abgabe des Therapeutikums nur sinnvoll, wenn der Patient durch das Therapiegerät einatmet. Daher ist in interaktiven Inhalatoren ein Sensor vorgesehen, welcher einen Atemluftstrom des Patienten registriert. In Reaktion auf die Registrierung des Atemluftstroms wird dann das Therapeutikum freigesetzt. Besondere Formen von Inhalatoren sind z.B. MDI's ("metered dose inhaler"), welche zur Inhalation kleiner Dosen eines Therapeutikums mit Treibgasunterstützung dienen, oder DPl's ("dry powder inhaler"), welche zur Inhalation von Therapeutika in Pulverform eingesetzt werden.

Parameter eines dabei durch die Therapieeinheit anzuwendenden Therapieprogramms können auf einer Speichereinheit hinterlegt sein, welche in der Regel Bestandteil einer Steuerungseinheit ist. Die Steuerungseinheit ist eingerichtet, die Parameter aus der Speichereinheit auszulesen und zur Ansteuerung der Therapieeinheit zu verwenden.

Bei vielen interaktiven Therapiegeräten hängt der Therapieerfolg mehr oder weniger stark davon ab, ob die Interaktion des Patienten mit der Therapiegerät gewissen Anforderungen entspricht. Um die Übereinstimmung der tatsächlichen Interaktion des Patienten mit vorgegebenen Anforderungen zu kontrollieren, weisen manche moderne Therapiegeräte eine Kommunikationsschnittstelle auf, über welche Messwerte des wenigstens einen Sensors ausgegeben werden können. Die Ausgabe kann beispielsweise an externe Datenverarbeitungsgeräte erfolgen, welche die Messdaten hinsichtlich verschiedener Kriterien auswerten und die Ergebnisse visualisieren. Aus der entsprechenden Visualisierung können der Patient und/oder eine behandelnde Fachkraft Hinweise ableiten, wie das Therapieverhalten des Patienten ggf. verbessert werden kann.

Ein weiterer Nutzen der Datenausgabe besteht darin, dass das Patientenverhalten beispielsweise bei der Durchführung von klinischen Studien mit einem Therapeutikum herangezogen werden kann, um die Aussagekraft der Studie zu erhöhen. Beispielsweise können bei einer randomisierten Doppel-Blind-Studie über die Wirksamkeit eines Therapeutikums Messwerte über das Verhalten der Versuchspersonen erfasst werden, und die Ergebnisse von Versuchspersonen, welche vorgegebene Verhaltensweisen bei der Anwendung des Therapiegeräts nicht einhalten, können bei der Auswertung der Studie ausgeschlossen werden.

Ein entsprechendes Therapiegerät ist beispielsweise aus der EP3375473A1 bekannt. Das dort beschriebene Therapiegerät ist eingerichtet, Messdaten an ein externes Steuergerät, beispielsweise ein Mobiltelefon, zu übertragen. Über das Steuergerät erhält der Patient ein Feedback bezüglich seines Therapieverhaltens.

Die Kommunikationsschnittstelle kann es weiterhin einem Steuergerät ermöglichen, das Verhalten des Therapiegeräts zu beeinflussen. So ist beispielsweise das Therapiegerät des oben genannten Standes der Technik so eingerichtet, dass die Art der Datenausgabe durch das externe Steuergerät beeinflusst werden kann.

Die Möglichkeit, das Verhalten eines Therapiegeräts über eine Kommunikationsschnittstelle zu beeinflussen, wirft allerdings diverse Probleme auf. Für die Zulassung eines Therapiegeräts ist es erforderlich, die ordnungsgemäße Funktion des Therapiegeräts unter allen Umständen sicherzustellen. Wenn das Therapiegerät über eine Kommunikationsschnittstelle durch andere Geräte beeinflusst werden kann, so müssen diese weiteren Geräte, oder ggf. auf diesen Geräten laufende Software, ebenfalls einem entsprechenden Zulassungsverfahren unterzogen werden. Dies ist aufwendig und zum Teil nahezu unmöglich, wie z.B. im Falle von handelsüblichen Mobiltelefonen oder Tablet-Computern mit offenem Betriebssystem.

Es besteht daher eine Aufgabe der Erfindung darin, ein Therapiegerät bereitzustellen, welches hinsichtlich der beschriebenen Problematik verbessert ist.

Eine weitere Aufgabe der Erfindung besteht darin, die Nutzungsmöglichkeiten eines interaktiven Therapiegeräts zu erweitern.

Zumindest eine der genannten Aufgaben wird gemäß eines ersten Aspekts der Erfindung gelöst durch ein interaktives Therapiegerät nach Anspruch 1.

Durch die entsprechende Ausführung des interaktiven Therapiegeräts können die Vorteile der Datenausgabe über die Kommunikationsschnittstelle ausgenutzt werden, ohne die Funktion des Therapiegeräts durch eine unzulässige Beeinflussung zu beeinträchtigen. Die Kommunikationsschnittstelle kann vorzugsweise eine drahtlose Schnittstelle sein oder eine solche umfassen. Dadurch kann das Therapiegerät besonders unkompliziert verwendet werden. Als drahtlose Schnittstelle kommen beispielsweise eine WiFi-Schnittstelle, eine Mobilfunk-Schnittstelle, und/oder eine Bluetooth-Schnittstelle in Frage. In einer bevorzugten Ausführung eines interaktiven Therapiegeräts kann die Kommunikationsschnittstelle einen Konfigurationsspeicher umfassen, auf welchem Konfigurationsdaten zur Herstellung einer Verbindung mit einem Netzwerk hinterlegt oder hinterlegbar sind.

Hierbei kann der Konfigurationsspeicher vorzugsweise von der Speichereinheit der Steuerungseinheit logisch oder physisch getrennt sein. Auf diese Weise kann sichergestellt sein, dass die Integrität von in der Speichereinheit hinterlegten therapierelevanten Daten nicht kompromittiert wird, wenn Konfigurationsdaten in dem Konfigurationsspeicher hinterlegt oder geändert werden.

Weiterhin kann ein interaktives Therapiegerät eine sekundäre Funktionseinheit umfassen, und die sekundäre Funktionseinheit kann über die Kommunikationsschnittstelle aktiviert und/oder deaktiviert werden.

Als eine sekundäre Funktionseinheit wird im Sinne der Erfindung eine Funktionseinheit des Therapiegeräts bezeichnet, deren Funktion für die therapeutische Funktion des Therapiegeräts nicht erforderlich ist.

Wenigstens eine weitere der genannten Aufgaben wird gemäß eines zweiten Aspekts der Erfindung gelöst durch ein vernetztes Therapiesystem, umfassend: wenigstens ein Therapiegerät; einen Datenserver; und wenigstens ein Visualisierungsgerät; wobei das Therapiegerät ein interaktives Therapiegerät gemäß den vorangehenden Ausführungen ist; der Datenserver eingerichtet ist, Messdaten von dem wenigstens einen Therapiegerät über einen ersten Datenkanal zu empfangen und zu speichern; und das Visualisierungsgerät eingerichtet ist, Messdaten von dem Datenserver über einen zweiten Datenkanal zu empfangen und diese zu visualisieren; wobei der zweite Datenkanal unabhängig von dem ersten Datenkanal ist.

In einem entsprechend ausgeführtem Therapiesystem ist jederzeit sichergestellt, dass der Datenserver vollständige Messdaten des Therapiegeräts erhält, ohne dass die Datenübertragung von dem Therapiegerät zu dem Datenserver durch Wechselwirkungen mit der Datenübertragung zwischen dem Datenserver und dem Visualisierungsgerät beeinträchtigt wird. Es kann somit eine vollwertige Dokumentation der Messdaten des Therapiegeräts erfolgen, beispielsweise zur Durchführung von klinischen Studien über ein spezielles Therapeutikum, ein spezielles Dosierungsregime, oder eine neue Applikationsform, während gleichzeitig der Patient bzw. die Versuchsperson über das Visualisierungsgerät ein Feedback über die richtige Verwendung des Therapiegeräts erhält.

Die Datenübertragung über den ersten und/oder zweiten Datenkanal erfolgt dabei vorzugsweise verschlüsselt. Dies trägt der Tatsache Rechnung, dass therapiebezogene Daten grundsätzlich als eine besonders schützenswerte Form der persönlichen Daten anzusehen sind, auf welche ein unbefugter Zugriff ausgeschlossen sein sollte.

Insbesondere der erste Datenkanal kann beispielsweise über eine erste Transportverschlüsselung gesichert sein, deren Schlüssel fest, und vorzugsweise nicht auslesbar, in der Steuerungseinheit des Therapiegeräts einprogrammiert ist.

Der zweite Datenkanal kann dagegen über eine zweite Transportverschlüsselung gesichert sein, deren Schlüssel ebenfalls in der Steuerungseinheit des Therapiegerätes fest einprogrammiert ist, aber von dem Therapiegerät beispielsweise über eine geeignete Schnittstelle bereitgestellt wird, so dass ein Benutzer, der sich im Besitz des Therapiegeräts befindet, diesen Schlüssel auslesen und auf ein Visualisierungsgerät, welches sich ebenfalls im Besitz des Benutzers befindet, übertragen kann.

Der Schlüssel der zweiten Transportverschlüsselung kann auch auf andere Weise auslesbar am Therapiegerät hinterlegt sein, beispielsweise in Form eines aufgedruckten oder anderweitig aufgebrachten alphanumerischen Codes, oder eines Strich- oder QR-Codes. Dieser Code kann mit einem Visualisierungsgerät eingescannt werden, um den zweiten Datenkanal herzustellen.

Das wenigstens eine Visualisierungsgerät kann ein mobiles Datenverarbeitungsgerät sein, beispielsweise ein Mobiltelefon, ein Tablet-Computer oder ein persönlicher Datenassistent "PDA".

Ein vernetztes Therapiesystem kann nach einer weiteren Ausführungsform der Erfindung wenigstens ein Diagnosegerät umfassen, und der Datenserver kann eingerichtet sein, Diagnosedaten von dem Diagnosegerät zu empfangen.

Dabei kann der Datenserver eingerichtet ist sein, Diagnosedaten von dem wenigstens einen Diagnosegerät über einen dritten Datenkanal zu empfangen, welcher von dem ersten und/oder dem zweiten Datenkanal unabhängig ist. Der dritte Datenkanal kann dabei ebenfalls verschlüsselt sein.

Das wenigstens eine Diagnosegerät kann ein Spirometer sein oder umfassen. Spirometer dienen dazu, charakteristische Daten der Atemfunktion eines Patienten zu bestimmen. Dies können das Atemvolumen, die Atemfrequenz, der mittlere Luftdurchsatz, und/oder der Spitzenwert des Luftdurchsatzes sein.

Entsprechende Diagnosedaten können in Verbindung mit den Messwerten des Therapiegeräts Aufschluss über einen Therapieerfolg geben, wenn beispielsweise über das Therapiegerät ein Therapeutikum verabreicht wird, welches die Atemfunktion des Patienten verbessern soll.

Ebenso kann das wenigstens eine Diagnosegerät ein Fitnesstracker sein oder einen solchen umfassen. Als Fitnesstracker werden im Sinne der Erfindung tragbare Geräte verstanden, welche grundlegende Aktivitätsdaten eines Benutzers messen und aufzeichnen, wie z.B. Herz- und/oder Atemfrequenz, Schrittfrequenzen, Bewegungsmuster, Körpertemperatur, oder ähnliches.

Diagnosedaten eines Fitnesstrackers können in Verbindung mit Messdaten des Therapiegeräts Rückschlüsse dazu ermöglichen, ob ein Patient während einer Therapie ein angemessenes Bewegungsverhalten zeigt, und/oder ob ein zu verabreichendes Therapeutikum beispielsweise direkte Auswirkungen auf Vitalparameter wie Puls oder Körpertemperatur hat.

Grundsätzlich können in einem vernetzten Therapiesystem gemäß der Erfindung auch mehrere Therapiegeräte mit oder ohne zusätzliche Diagnosegeräte vorgesehen sein. Dabei wird in der Regel ein Therapiegerät, welches vorzugsweise zur Benutzung durch lediglich einen einzigen individuellen Patienten vorgesehen ist, als ein "primary device" fungieren, welches sie Identität eines Datensatzes auf dem Datenserver vorgibt, während alle weiteren Therapie- oder Diagnosegeräte als "secondary device" fungieren, welche permanent oder temporär einem bestimmten "primary device" zugeordnet werden.

Die Erfindung wird nachfolgend anhand einiger beispielhafter Darstellungen näher erläutert, wobei die beschriebenen Ausführungsbeispiele lediglich zum besseren Verständnis der Erfindung betragen sollen, ohne diese einzuschränken.

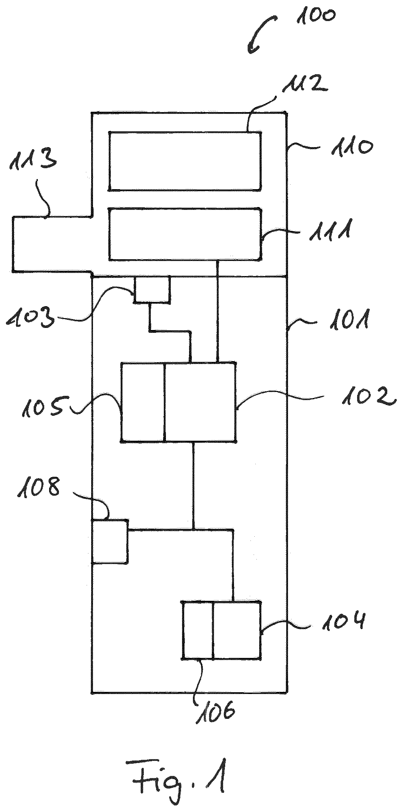

In Figur 1 ist ein interaktives Medizingerät 100 dargestellt, bei dem es sich im dargestellten Beispiel um einen Vernebler handelt. Der Vernebler umfasst eine Basis 101, welche im Wesentlichen eine Steuerungseinheit 102, einen Sensor 103, und eine Kommunikationsschnittstelle 104 umfasst. Die Steuerung 102 umfasst ein Speicherelement 105, welches beispielsweise als FLASH- oder EEPROM-Speicher ausgeführt sein kann.

Das Medizingerät umfasst weiterhin einen Kopf 110, welcher auf die Basis 101 aufgesetzt ist. Der Kopf 110 beinhaltet eine Therapieeinheit 111, hier in Form eines Aerosolgenerators, und eine Dosiereinheit 112, über welche ein Therapeutikum dosiert zugeführt werden kann. Weiterhin umfasst der Kopf ein Mundstück 113.

Um das Therapiegerät zu benutzen, atmet ein Patient durch das Mundstück 113 ein und erzeugt dabei einen Luftstrom durch die Therapieeinheit 111. Dieser Luftstrom wird durch den Sensor 103 als eine Interaktion eines Benutzers mit dem Therapiegerät 100 registriert.

Der Sensor 103 erzeugt ein Signal, welches vor der Steuerung 102 ausgewertet wird. Dazu vergleicht die Steuerung 102 beispielsweise eine Intensität, ein Timing, oder andere Eigenschaften des Signals mit Referenzwerten, welche in dem Speicherelement 105 hinterlegt sind. Entspricht das Signal bestimmten Vorgaben, so aktiviert die Steuerungseinheit 102 die Therapieeinheit 111.

Die Therapieeinheit 111 kann die Dosiereinheit 112 veranlassen, eine vorgegebene Menge eines Therapeutikums an die Therapieeinheit 111 abzugeben, welche dann beispielsweise als Aerosol in den vom Patienten eingeatmeten Luftstrom eindosiert wird.

Die Dosiereinheit kann auch als manuelle Dosiereinheit ausgeführt sein, welche vor dem Einatmen durch das Mundstück 113 manuell zu betätigen ist.

Neben vorstehend genannten Referenzwerten sind auf der Speichereinheit 105 weitere Informationen über ein Therapieprogramm hinterlegt, welches durch die Therapieeinheit 111 auszuführen ist. Dies können z.B. eine Betriebsfrequenz eines Ultraschallverneblers, eine vorgesehene Dauer einer Aktivierung des Aerosolgenerators, oder ähnliche Daten sein. Der korrekte Inhalt der Speichereinheit 105 ist daher für eine wirksame und sichere Anwendung des Therapiegeräts durch einen Patienten essentiell.

Die Kommunikationsschnittstelle 104 dient dazu, Messdaten des Sensors 103, und ggf. Messdaten weiterer nicht dargestellter Sensoren, auszugeben, beispielsweise im Rahmen eines vernetzten Therapiesystems. Um eine unzulässige Manipulation der auf dem Speicherelement 105 hiterlegten Informationen ist die Steuerungseinheit 102 eingerichtet, einen schreibenden Zugriff auf das Speicherelement 105 über die Kommunikationsschnittstelle 104 zu verhindern. Dazu kann das Speicherelement 105 als "read only"-Speicher ausgeführt sein, oder einen Schreibschutz aufweisen, welcher nur im Rahmen eines Service-Eingriffs in die Basis aufgehoben werden kann.

Das Medizingerät 100 kann eine weitere, sekundäre Funktionseinheit 108 aufweisen, deren Funktion nicht unmittelbar therapierelevant ist. Es ist daher möglich, eine Aktivierung und/oder Deaktivierung der sekundären Funktionseinheit 108 über die Kommunikationsschnittstelle 104 zuzulassen, ohne dabei eine Kompromittierung der Funktionssicherheit des Medizingeräts 100 zu riskieren. Die sekundäre Funktionseinheit 108 kann beispielsweise ein Display und/oder ein anderes Signalausgabegerät umfassen, über welches die Steuerungseinheit 102 einem Benutzer des Medizingeräts 100 ein direktes Feedback vermitteln kann.

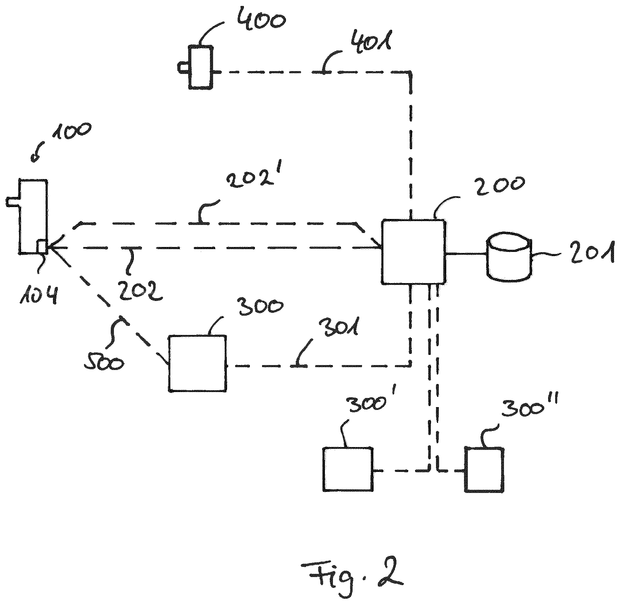

Ein vernetztes Therapiesystem ist in Figur 2 dargestellt. Es umfasst das Therapiegerät 100 sowie einen Datenserver 200, welcher mit einer Datenbank 201 verbunden ist.

Zwischen der Kommunikationsschnittstelle 104 und dem Datenserver 200 ist ein erster Datenkanal 202 aufgebaut. Der Datenkanal 202 kann über nicht dargestellte Zwischenstationen verlaufen, beispielsweise WiFi-Router, Mobilfunkrelais, und/oder das Internet.

Zur Herstellung des Datenkanals 202 weist die Kommunikationsschnittstelle 104 einen Konfigurationsspeicher 106 auf, in dem Konfigurationsdaten zur Herstellung des Datenkanals 202 hinterlegt sind. Diese Konfigurationsdaten können insbesondere Zugangsdaten zu einem Netzwerk umfassen, beispielsweise Anmeldedaten für einen WiFi-Router oder Nutzeridentifikationsdaten für ein Mobilfunknetz. Die Konfigurationsdaten können dabei statisch oder veränderbar sein. Wenn die Kommunikationsschnittstelle 104 eine WiFi-Schnittstelle umfasst, so können die Anmeldedaten für ein Netzwerk, mit welchem sich die Kommunikationsschnittstelle 104 verbinden soll, beispielsweise über eine separate Verbindung, z.B. eine Bluetooth-Verbindung, an die Kommunikationsschnittstelle 104 übertragen und dann in dem Konfigurationsspeicher 106 abgelegt werden. Sollten sich die Zugangsdaten ändern, können die hinterlegten Konfigurationsdaten auf gleichem Weg aktualisiert werden. Für eine Mobilfunkschnittstelle hingegen kann der Konfigurationsspeicher 106 statisch ausgeführt sein, z.B. in Form einer "SIM"-Karte.

Die Steuerungseinheit 102 ist eingerichtet, die Herstellung des Datenkanals 202, ggf. nach erstmaliger Vorgabe der Anmeldedaten, automatisch herzustellen, und Mess-und/oder Betriebsdaten automatisch an den Datenserver 200 zu übertragen. Durch diese automatische Übertragung ist sichergestellt, dass therapierelevante Daten nicht verloren gehen. Für den Fall einer fehlenden oder unzureichenden Datenverbindung kann das Therapiegerät 100 über einen nicht dargestellten Zwischenspeicher verfügen, um die entsprechenden Daten zu speichern, bis wieder eine Datenverbindung zu dem Datenserver hergestellt werden kann.

Die Steuerungseinheit 102 kann auch eingerichtet sein, in einem ersten Betriebsmodus und in einem zweiten Betriebsmodus zu operieren, wobei in dem ersten Betriebsmodus ein schreibender Zugriff auf das Speicherelement 105 ausgeschlossen ist, und dem zweiten Betriebsmodus ein schreibender Zugriff auf das Speicherelement 105 zugelassen ist. Um auch hierbei eine unzulässige Manipulation der hinterlegten Informationen zu verhindern, ist die Steuerungseinheit 102 eingerichtet, in dem zweiten Betriebsmodus der Kommunikationsschnittstelle 104 nur eine Verbindung mit einem vorgegebenen proprietären Netzwerk herzustellen, welches beispielsweise von einem speziellen Router aufgebaut wird. Die Steuerungseinheit 102 kann jeweils prüfen, ob das proprietäre Netzwerk vorhanden ist, und nur dann den zweiten Betriebsmodus aktivieren, wenn das proprietäre Netzwerk gefunden wird. Eine Datenverbindung über das proprietäre Netzwerk ist durch die Linie 202' dargestellt. Wird das proprietäre Netzwerk hingegen nicht aufgefunden, so versucht die Steuerungseinheit, mit den in dem Konfigurationsspeicher 106 hinterlegten Verbindungsdaten eine Datenverbindung zu dem Datenserver 200 aufzubauen. Durch diese besondere Ausführung ist es möglich, beispielsweise im Rahmen von Studien Betriebsparameter des Therapiegeräts 100 anzupassen, ohne jeweils direkt auf das Therapiegerät 100 zugreifen zu müssen. Das Therapiesystem umfasst weiterhin ein Visualisierungsgerät 300, welches über einen zweiten Datenkanal 301 mit dem Datenserver 200 verbunden ist. Im dargestellten Beispiel ist das Visualisierungsgerät 300 ein Mobiltelefon, und der zweite Datenkanal 301 umfasst eine Mobilfunkverbindung.

Das Visualisierungsgerät 300 kann alternativ auch ein handelsüblicher Personal Computer (PC) sein, in diesem Fall kann der zweite Datenkanal 301 eine Internetverbindung beinhalten.

Das Visualisierungsgerät 300 umfasst Hardwarekomponenten und Softwarekomponenten. Die Softwarekomponenten sind dabei eingerichtet, über den zweiten Datenkanal 301 die Verbindung mit dem Datenserver 300 aufzubauen, um von dem Therapiegerät 100 an den Datenserver 200 übertragene Messdaten abzufragen und anzuzeigen.

Die Art der Anzeige kann dabei an verschiedene Nutzungsarten des Visualisierungsgeräts 300 in dem Therapiesystem angepasst sein. Wenn beispielsweise das Visualisierungsgerät 300 dem Benutzer des Therapiegeräts 100 zugeordnet ist, so kann die Art der Anzeige so eingerichtet sein, dass der Benutzer aus der Anzeige Hinweise für mögliche Verbesserungen an seinem Nutzungsverhalten ableiten kann. Ist hingegen das Visualisierungsgerät einem behandelnden Arzt des Benutzers zugeordnet, so kann die Anzeige beispielsweise so eingerichtet sein, dass der Arzt aus der Anzeige Informationen über die Einhaltung eines Therapieplans durch den Benutzer, und/oder über Therapiefortschritte ableiten kann.

Das Visualisierungsgerät 300 wird in den meisten Fällen kein qualifiziertes Medizingerät sein. Um eine unzulässige Beeinflussung des Therapiergeräts 100 durch das Visualisierungsgerät zu verhindern, ist der zweite Datenkanal 301 vorzugsweise als quasiunidirektionaler Datenkanal ausgeführt. Unter einem quasi-unidirektionalen Datenkanal wird hierbei ein Datenkanal verstanden, über den das Visualisierungsgerät 300 nur vordefinierte Anforderungssignale an den Datenserver 200 senden kann, während der Datenserver über den Datenkanal 301 beliebige Daten an das Visualisierungsgerät 300 senden kann.

Das Therapiesystem kann weiterhin ein Diagnosegerät 400 umfassen, welches über einen dritten Datenkanal 401 mit dem Datenserver verbunden ist. Unter dem Begriff "Diagnosegerät" wird hier ein Gerät verstanden, welches im weitesten Sinne diagnoserelevante Messdaten eines Patienten erheben kann.

Bei dem Diagnosegerät 400 kann es sich beispielsweise um ein Spirometer handeln. Ein Spirometer dient dazu, Kennwerte der Atemfunktion eines Patienten zu ermitteln, beispielsweise das Atemvolumen, den mittleren Volumenstrom beim Ein- und/oder Ausatmen, oder den maximalen Volumenstrom beispielsweise bei einem Hustenstoß. Der Verlauf solcher Kennwerte über die Dauer einer Therapie kann einem behandelnden oder begleitenden Arzt Hinweise über den Therapieerfolg geben.

Bei dem Diagnosegerät 400 kann es sich auch um einen Fitnesstracker handeln. Fitnesstracker sind am Körper getragene Messgeräte, welche regelmäßig Daten wie Pulsschlag, Atemfrequenz, und/oder Bewegungsprofil des Benutzers ermitteln. Solche Messdaten können ebenfalls für einen behandelnden oder begleitenden Arzt interessant sein, um das Allgemeinverhalten eines Patienten während einer Therapie zu beurteilen.

Messdaten eines Fitnesstrackers können auch für andere in die Therapie eingebundene Fachkräfte interessant sein, wie z.B. Physiotherapeuten und/oder Pflegekräfte.

Auch wenn das Therapiesystem in Figur 2 nur mit einem einzigen Diagnosegerät 400 dargestellt ist, kann ein Therapiesystem selbstverständlich auch mehrere Visualisierungsgeräte und/oder Diagnosegeräte beinhalten.

Um einen unbefugten Zugriff auf Messdaten zu verhindern, die über die Datenkanäle 202, 301, 401 übertragen werden, sind die jeweiligen Datenkanäle 202, 301, 401 vorzugsweise verschlüsselt.

Für den Datenkanal 202 kann beispielsweise eine symmetrische Transportverschlüsselung mit einem geheimen Schlüssel vorgesehen sein, welcher nur der Steuerungseinheit 102 des Therapiegeräts 100 sowie dem Datenserver 200 bekannt sind. Dieser Schlüssel kann in der Steuerungseinheit 102 so hinterlegt sein, dass er nicht bzw. nicht ohne weiteres auslesbar ist. In der Datenbank 201 kann ein Datensatz für das Therapiegerät 100 hinterlegt sein, welcher ebenfalls den Schlüssel enthält.

Bei Herstellung des Datenkanals 202 zwischen dem Therapiegerät 100 und dem Datenserver 200 kann das Therapiegerät zunächst eine Kennung an den Datenserver 200 übertragen. Der Datenserver 200 ermittelt mit der Kennung den zu dem Therapiegerät 100 gehörenden Datensatz in der Datenbank 201 und liest daraus den Schlüssel aus. Das Therapiegerät 100 kann nun Messwerte verschlüsselt an den Datenserver 200 übertragen, der Datenserver 200 entschlüsselt die Daten, und legt sie in dem zum Therapiegerät 100 gehörenden Datensatz ab.

Das Therapiegerät 100 fungiert als primäres Gerät in dem Therapiesystem, d.h. zwischen dem Therapiegerät 100 und dem dazugehörigen Datensatz in der Datenbank 201 existiert eine vorgegebene feste Zuordnung.

Das Visualisierungsgerät 300 fungiert in dem Therapiesystem als ein sekundäres Gerät. Dies bedeutet, dass das Visualisierungsgerät 300 temporär oder permanent wenigstens einem bestimmten Therapiegerät zugeordnet sein muss, hier dem Therapiegerät 100.

Die Zuordnung des Visualisierungsgeräts 300 zu dem Therapiegerät 100 kann z.B. so erfolgen, dass zwischen dem Therapiegerät 100 und dem Visualisierungsgerät 300 temporär eine Datenverbindung 500 aufgebaut wird, beispielsweise eine Bluetooth-Verbindung, über welche das Therapiegerät seine Kennung und einen Aktivierungscode an das Visualisierungsgerät 300 überträgt. Der Aktivierungscode wird parallel über den Datenkanal 202 an den Datenserver 200 übertragen. Das Visualisierungsgerät 300 kann dann den Datenkanal 301 zu dem Datenserver 200 herstellen, beispielsweise durch Aufrufen einer bestimmten Internetadresse, und sich durch Angabe der Kennung des Therapiegeräts 100 und des Aktivierungscodes dort anmelden. Das Visualisierungsgerät 300 ist nun dem Therapiegerät 100 zugeordnet, so dass der Datenserver Messwerte des Therapiegeräts 100, die in dem entsprechenden Datensatz abgelegt sind, an das Visualisierungsgerät 300 übermittelt.

Das Visualisierungsgerät 300 kann zusätzlich zu einer reinen Visualisierung von Messwerten zusätzliche Funktionen erfüllen. So kann das Visualisierungsgerät 300 eine Kalenderfunktion ausführen, um einen Patienten rechtzeitig an notwendige Anwendungen des Therapiegeräts 100 zu erinnern. Das Visualisierungsgerät kann auch eine Motivierungsfunktion für einen Patienten ausführen, beispielsweise in dem eine Erfüllungsrate von vorgesehenen Anwendungen des Therapiegeräts ermittelt und angezeigt wird, und indem zusätzlich in Abhängigkeit von der Erfüllungsrate mahnende, anspornende, und/oder belohnende Mitteilungen ausgegeben werden.

Ebenso kann der Patient über das Visualisierungsgerät 300 ein unmittelbares Feedback über seine Verwendung des Therapiegeräts 100 erhalten, um beispielsweise eine korrekte oder eine fehlerhafte Verwendung zu erkennen. Auf diese Weise kann der Patient in der korrekten Anwendung des Therapiegeräts 100 trainiert werden. Das Feedback kann einfache optische, akustische, und/oder haptische Signale umfassen, und/oder grafische Darstellungen von Betriebsparametern des Therapiegeräts.

Das Feedback kann über die Datenkanäle 202 und 301 erfolgen, wenn diese eine angemessene Datenübertragungsrate aufweisen. Alternativ kann auch für die Feedbackfunktion die zusätzliche Datenverbindung 500 genutzt werden.

Die zusätzliche Datenverbindung 500 kann auch genutzt werden, um im Falle einer fehlenden Datenverbindung des Therapiegeräts 100 mit dem Datenserver 200 Mess-und/oder Betriebsdaten des Therapiegeräts 100 an das Visualisierungsgerät 300 zu senden, damit diese dort temporär oder permanent gespeichert werden können. Auf diese Weise kann ein Patient auch trotz länger unterbrochener Verbindung zum Datenserver 200 zumindest auf eine Visualisierung der Daten zugreifen, die während der Unterbrechung der Datenverbindung auf dem Visualisierungsgerät gespeichert wurde.

Das Visualisierungsgerät 300 kann mehr als einem Therapiegerät zugeordnet sein. Wenn das Visualisierungsgerät 300 beispielsweise ein Computer in einer Arztpraxis oder in einem Krankenhaus ist, so kann das Visualisierungsgerät nacheinander Kennungen und Aktivierungscodes von mehreren Therapiegeräten empfangen, und sich damit bei dem Datenserver 200 anmelden.

Falls in einem Therapiesystem mehrere Visualisierungsgeräte eingebunden werden sollen, so kann nach der Zuordnung des ersten Visualisierungsgeräts 300 eine Zuordnung weiterer Visualisierungsgeräte 300', 300" auch ohne direkte Datenverbindung zwischen dem Therapiegerät 100 und den weiteren Visualisierungsgeräten 300', 300' erfolgen. Hierzu kann sich der Verwender des Therapiegeräts 100 an dem ersten Visualisierungsgerät 300 autorisieren, und dann über den Datenkanal 301 einen temporären Freigabecode erhalten, welcher er dann an einen Benutzer des weiteren Visualisierungsgeräts 300', 300" übermitteln kann. Die Übermittlung kann über einen separaten Datenkanal, beispielsweise per Email, SMS, oder andere Kanäle erfolgen. Der Benutzer des weiteren Visualisierungsgeräts 300', 300" kann sich dann mit diesem Freigabecode ebenfalls in dem Therapiesystem anmelden, wodurch das weitere Visualisierungsgerät 300', 300" dem Therapiegerät 100 zugeordnet wird.

Bei einer solchen manuellen Zuordnung weiterer Visualisierungsgeräte kann dem Verwender des Therapiegeräts 100 über eine Möglichkeit gegeben werden, Art und Umfang der Daten auszuwählen, welche an die weiteren Visualisierungsgeräte 300', 300" übermittelt werden.

Kennungen und Aktivierungscodes der Therapiegeräte können in dem Visualisierungsgerät 300 gespeichert werden, so dass nicht für jede Anmeldung des Visualisierungsgeräts 300 eine neue Verbindung zu dem jeweiligen Therapiegerät hergestellt werden muss. So kann beispielsweise ein Arzt im Falle einer telefonischen oder anderweitigen Rückfrage eines Patienten ohne unmittelbaren Zugriff auf das jeweilige Therapiegerät auf die aktuellen Messdaten dieses Therapiegeräts zugreifen. Hierzu kann durch eine auf dem Visualisierungsgerät 300 laufende Software ein Auswahlmenü angezeigt werden, in welchem der Arzt auswählen kann, von welchem Therapiegerät er Messdaten angezeigt bekommen möchte.

Ähnlich wie das Visualisierungsgerät 300 fungiert auch das Diagnosegerät 400 als sekundäres Gerät, es kann also nur mit dem Datenserver 200 kommunizieren, wenn es einem bestimmten Therapiegerät zugeordnet ist, hier wieder dem Therapiegerät 100.

Die Anmeldung des Diagnosegeräts 400 bei dem Datenserver 200 kann ähnlich geschehen wie die Anmeldung des Visualisierungsgeräts 300, sie wird daher nicht noch einmal im Detail beschrieben.

Je nach Art des Diagnosegeräts 400 kann dieses nur mit einem einzigen Therapiegerät oder auch mit mehreren Therapiegeräten verknüpft werden. So wird beispielsweise ein Fitnesstracker nur einem einzigen Therapiegerät verknüpft werden, während ein stationäres Diagnosegerät naturgemäß eher mit mehreren Therapiegeräten verknüpft sein wird.

Neben der beschriebenen therapiebegleitenden Anwendung eines vernetzten Therapiesystems kann ein solches System auch im Rahmen von klinischen Studien vorteilhaft eingesetzt werden. So kann eine große Anzahl von Testpersonen mit Therapiegräten 100 ausgestattet werden, und die Messwerte der Therapiegeräte werden automatisch an den Datenserver 200 übertragen. Messwerte von Diagnosegeräten 400 können ebenfalls automatisch an den Datenserver 200 übertragen werden, so dass alle zur Auswertung der Studie erforderlichen Daten unmittelbar zur Verfügung stehen.

Über ein entsprechendes Therapiesystem kann auch eine Überprüfung erfolgen, welche Arten von Rückmeldungen durch Visualisierungsgeräte 300 an Patienten am ehesten geeignet sind, um eine hohe Erfüllungsrate bei der Verwendung des Therapiegeräts 100 zu erreichen. Dazu können einzelne Visualisierungsgeräte 300 über den Datenkanal 301 unterschiedliche Arten von motivierenden Botschaften erhalten und diese unter vorgegebenen Bedingungen ausgeben. Über die Messwerte der Therapiegeräte, denen die Visualisierungsgeräte zugeordnet sind, lässt sich dann der Einfluss der Botschaften auf den Erfüllungsgrad ermitteln.

Die Erfindung wird durch die angehängten Ansprüche definiert.

## Patentansprüche

1. Interaktives Therapiegerät (100), das als Vernebler ausgebildet ist, umfassend
- eine Basiseinheit (101) mit wenigstens einem Sensor (103), einer Steuerungseinheit (102), sowie einer Kommunikationsschnittstelle (104); und einen Kopf (110), welcher auf die Basiseinheit 101 aufgesetzt ist, mit einer Therapieeinheit (111), einer Dosiereinheit (112) und einem Mundstück (113);
wobei die Therapieeinheit (111) ein Aerosolgenerator ist;
wobei der wenigstens eine Sensor (103) eingerichtet ist, eine Interaktion eines Benutzers mit dem Therapiegerät (100) zu registrieren, und die Steuerungseinheit (102) eingerichtet ist, bei Feststellung einer Interaktion des Benutzers mit dem Therapiegerät (100) die Therapieeinheit (111) zu aktivieren; wobei ferner die Steuerungseinheit (102)
eine Speichereinheit (105) umfasst, auf welcher Informationen über ein durch die Therapieeinheit (111) durchzuführendes Therapieprogramm hinterlegt sind, und die Steuerungseinheit (102) eingerichtet ist, die Therapieeinheit (111) entsprechend der auf der Speichereinheit (105) hinterlegten Informationen zu aktivieren; und
wobei die Steuerungseinheit (102) ferner eingerichtet ist, Messdaten des wenigstens einen Sensors über die Kommunikationsschnittstelle (104) auszugeben;
wobei die Steuerungseinheit (102) eingerichtet ist, einen schreibenden Zugriff auf die auf der Speichereinheit (105) hinterlegten Informationen über die Kommunikationsschnittstelle (104) zu verhindern.

2. Interaktives Therapiegerät (100) nach Anspruch 1, **dadurch gekennzeichnet, dass** die Kommunikationsschnittstelle (104) eine drahtlose Schnittstelle ist oder umfasst.

3. Interaktives Therapiegerät (100) nach Anspruch 2, **dadurch gekennzeichnet, dass** die Kommunikationsschnittstelle (104) eine WiFi-Schnittstelle umfasst.

4. Interaktives Therapiegerät (100) nach Anspruch 2 oder 3, **dadurch gekennzeichnet, dass** die Kommunikationsschnittstelle (104) eine Mobilfunk-Schnittstelle umfasst.

5. Interaktives Therapiegerät (100) nach einem der Ansprüche 2 bis 4, **dadurch gekennzeichnet, dass** die Kommunikationsschnittstelle (104) eine Bluetooth-Schnittstelle umfasst.

6. Interaktives Therapiegerät (100) nach einem der Ansprüche 2 bis 5, **dadurch gekennzeichnet, dass** die Kommunikationsschnittstelle (104) einen Konfigurationsspeicher umfasst, auf welchem Konfigurationsdaten zur Herstellung einer Verbindung mit einem Netzwerk hinterlegt oder hinterlegbar sind.

7. Interaktives Therapiegerät (100) nach einem der vorangehenden Ansprüche, **dadurch gekennzeichnet, dass** das Therapiegerät (100) eine sekundäre Funktionseinheit (108) umfasst, und dass die sekundäre Funktionseinheit über die Kommunikationsschnittstelle (104) aktiviert und/oder deaktiviert werden kann.

8. Vernetztes Therapiesystem, umfassend:
- wenigstens ein Therapiegerät (100),
- einen Datenserver (200), und
- wenigstens ein Visualisierungsgerät (300),
wobei das Therapiegerät (100) ein interaktives Therapiegerät (100) gemäß einem der vorangehenden Ansprüche ist,
der Datenserver (200) eingerichtet ist, Messdaten von dem wenigstens einen Therapiegerät (100) über einen ersten Datenkanal zu empfangen und zu speichern, und
das Visualisierungsgerät (300) eingerichtet ist, Messdaten von dem Datenserver (200) über einen zweiten Datenkanal (301) zu empfangen und diese zu visualisieren, wobei der zweite Datenkanal (301) unabhängig von dem ersten Datenkanal ist.

9. Vernetztes Therapiesystem nach Anspruch 8, **dadurch gekennzeichnet, dass** die Datenübertragung über den ersten und/oder zweiten Datenkanal (301) verschlüsselt erfolgt.

10. Vernetztes Therapiesystem nach Anspruch 8 oder 9, **dadurch gekennzeichnet, dass** das wenigstens eine Visualisierungsgerät (300) ein mobiles Datenverarbeitungsgerät ist.

11. Vernetztes Therapiesystem nach einem der Ansprüche 8 bis 10, **dadurch gekennzeichnet, dass** das Therapiesystem wenigstens ein Diagnosegerät (400) umfasst, und dass der Datenserver (200) eingerichtet ist, Diagnosedaten von dem Diagnosegerät zu empfangen.

12. Vernetztes Therapiesystem nach Anspruch 11, **dadurch gekennzeichnet, dass** der Datenserver (200) eingerichtet ist, Diagnosedaten von dem wenigstens einen Diagnosegerät (400) über einen dritten Datenkanal (401) zu empfangen, welcher von dem ersten und/oder dem zweiten Datenkanal (301) unabhängig ist.

13. Vernetztes Therapiesystem nach Anspruch 11, **dadurch gekennzeichnet, dass** das wenigstens eine Diagnosegerät (400) ein Spirometer ist oder umfasst.

14. Vernetztes Therapiesystem nach Anspruch 11 oder 12, **dadurch gekennzeichnet, dass** das Diagnosegerät (400) ein Fitnesstracker ist oder einen solchen umfasst.

## Claims

1. An interactive therapy device (100) configured as a nebulizer, comprising a base unit (101) with at least one sensor (103), a control unit (102), and a communication interface (104); and
a head (110) mounted on the base unit (101), comprising a therapy unit (111), a dosing unit (112), and a mouthpiece (113);
wherein the therapy unit (111) is an aerosol generator;
wherein the at least one sensor (103) is configured to register a user's interaction with the therapy device (100), and the control unit (102) is configured to activate the therapy unit (111) upon registration of a user's interaction with the therapy device (100);
wherein the control unit (102) further comprises a storage unit (105) on which information regarding a therapy program to be performed by the therapy unit (111) is stored, and the control unit (102) is configured to activate the therapy unit (111) in accordance with the information stored on the storage unit (105); and
wherein the control unit (102) is further configured to output measurement data from the at least one sensor via the communication interface (104);
wherein the control unit (102) is configured to prevent write access to the information stored on the storage unit (105) via the communication interface (104).

2. An interactive therapy device (100) according to claim 1, **characterized in that** the communication interface (104) is or comprises a wireless interface.

3. An interactive therapy device (100) according to claim 2, **characterized in that** the communication interface (104) comprises a Wi-Fi interface.

4. Interactive therapy device (100) according to claim 2 or 3, **characterized in that** the communication interface (104) comprises a cellular interface.

5. Interactive therapy device (100) according to any one of claims 2 to 4, **characterized in that** the communication interface (104) comprises a Bluetooth interface.

6. An interactive therapy device (100) according to any one of claims 2 to 5, **characterized in that** the communication interface (104) comprises a configuration memory in which configuration data for establishing a connection to a network is stored or can be stored.

7. An interactive therapy device (100) according to any of the preceding claims, **characterized in that** the therapy device (100) comprises a secondary functional unit (108), and **in that** the secondary functional unit can be activated and/or deactivated via the communication interface (104).

8. A networked therapy system comprising:
- at least one therapy device (100),
- a data server (200), and
- at least one visualization device (300), wherein the therapy device (100) is an interactive therapy device (100) according to one of the preceding claims,
the data server (200) is configured to receive and store measurement data from the at least one therapy device (100) via a first data channel, and the visualization device (300) is configured to receive measurement data from the data server (200) via a second data channel (301) and to visualize them, wherein the second data channel (301) is independent of the first data channel.

9. A networked therapy system according to claim 8, **characterized in that** the data transmission via the first and/or second data channel (301) is encrypted.

10. A networked therapy system according to claim 8 or 9, **characterized in that** the at least one visualization device (300) is a mobile data processing device.

11. A networked therapy system according to any one of claims 8 to 10, **characterized in that** the therapy system comprises at least one diagnostic device (400), and **in that** the data server (200) is configured to receive diagnostic data from the diagnostic device.

12. A networked therapy system according to claim 11, **characterized in that** the data server (200) is configured to receive diagnostic data from the at least one diagnostic device (400) via a third data channel (401) that is independent of the first and/or second data channel (301).

13. A networked therapy system according to claim 11, **characterized in that** the at least one diagnostic device (400) is or comprises a spirometer.

14. A networked therapy system according to claim 11 or 12, **characterized in that** the diagnostic device (400) is or comprises a fitness tracker.

## Revendications

1. Appareil thérapeutique interactif (100) conçu comme nébuliseur, comprenant une unité de base (101) avec au moins un capteur (103), une unité de commande (102) et une interface de communication (104) ; et
une tête (110), qui est placée sur l'unité de base 101, avec une unité thérapeutique (111), une unité de dosage (112) et un embout buccal (113) ;
dans lequel l'unité thérapeutique (111) est un générateur d'aérosols ;
dans lequel le au moins un capteur (103) est configuré pour enregistrer une interaction d'un utilisateur avec l'appareil thérapeutique (100) et l'unité de commande (102) est configurée pour activer l'unité thérapeutique (111) lorsqu'une interaction de l'utilisateur avec l'appareil thérapeutique (100) est détectée ;
dans lequel l'unité de commande (102) comprend en outre une unité de mémoire (105) sur laquelle sont stockées des informations sur un programme thérapeutique à exécuter par l'unité thérapeutique (111), et l'unité de commande (102) est configurée pour activer l'unité thérapeutique (111) en fonction des informations stockées sur l'unité de mémoire (105) ; et
dans lequel l'unité de commande (102) est en outre configurée pour émettre des données de mesure du au moins un capteur via l'interface de communication (104) ; dans lequel l'unité de commande (102) est configurée pour empêcher l'accès en écriture aux informations stockées sur l'unité de mémoire (105) via l'interface de communication (104).

2. Appareil thérapeutique interactif (100) selon la revendication 1, **caractérisé en ce que** l'interface de communication (104) est ou comprend une interface sans fil.

3. Appareil thérapeutique interactif (100) selon la revendication 2, **caractérisé en ce que** l'interface de communication (104) comprend une interface WiFi.

4. Appareil thérapeutique interactif (100) selon la revendication 2 ou 3, **caractérisé en ce que** l'interface de communication (104) comprend une interface de téléphonie mobile.

5. Appareil thérapeutique interactif (100) selon l'une quelconque des revendications 2 à 4, **caractérisé en ce que** l'interface de communication (104) comprend une interface Bluetooth.

6. Appareil thérapeutique interactif (100) selon l'une quelconque des revendications 2 à 5, **caractérisé en ce que** l'interface de communication (104) comprend une mémoire de configuration sur laquelle des données de configuration peuvent être stockées ou peuvent être stockées pour établir une connexion à un réseau.

7. Appareil thérapeutique interactif (100) selon l'une quelconque des revendications précédentes, **caractérisé en ce que** l'appareil thérapeutique (100) comprend une unité fonctionnelle secondaire (108) et **en ce que** l'unité fonctionnelle secondaire peut être activée et/ou désactivée via l'interface de communication (104).

8. Système de thérapie en réseau, comprenant :
- au moins un appareil thérapeutique (100),
- un serveur de données (200), et
- au moins un appareil de visualisation (300),
dans lequel l'appareil thérapeutique (100) est un appareil thérapeutique interactif (100) selon l'une quelconque des revendications précédentes,
le serveur de données (200) est configuré pour recevoir et stocker les données de mesure d'au moins un appareil thérapeutique (100) via un premier canal de données, et
l'appareil de visualisation (300) est configuré pour recevoir des données de mesure du serveur de données (200) via un deuxième canal de données (301) et pour les visualiser, le deuxième canal de données (301) étant indépendant du premier canal de données.

9. Système de thérapie en réseau selon la revendication 8, **caractérisé en ce que** la transmission des données est cryptée via le premier et/ou le deuxième canal de données (301).

10. Système de thérapie en réseau selon la revendication 8 ou 9, **caractérisé en ce que** l'au moins un appareil de visualisation (300) est un appareil de traitement de données mobile.

11. Système de thérapie en réseau selon l'une quelconque des revendications 8 à 10, **caractérisé en ce que** le système de thérapie comprend au moins un appareil de diagnostic (400) et **en ce que** le serveur de données (200) est configuré pour recevoir des données de diagnostic de l'appareil de diagnostic.

12. Système de thérapie en réseau selon la revendication 11, **caractérisé en ce que** le serveur de données (200) est configuré pour recevoir des données de diagnostic à partir dudit au moins un appareil de diagnostic (400) via un troisième canal de données (401) qui est indépendant du premier et/ou du deuxième canal de données (301).

13. Système de thérapie en réseau selon la revendication 11, **caractérisé en ce que** l'au moins un appareil de diagnostic (400) est ou comprend un spiromètre.

14. Système de thérapie en réseau selon la revendication 11 ou 12, **caractérisé en ce que** l'appareil de diagnostic (400) est un tracker de fitness ou comprend un tel tracker.
